(19) Europäisches Patentamt
European Patent Office
Office européen des brevets

(11) **EP 3 925 521 A1**

(12) **EUROPEAN PATENT APPLICATION**

(43) Date of publication:
**22.12.2021   Bulletin 2021/51**

(51) Int Cl.:
**A61B 5/00** (2006.01)          **A61B 5/01** (2006.01)
**A61B 5/0205** (2006.01)      **A61B 5/113** (2006.01)
**A61B 5/1172** (2016.01)      **A61B 5/1171** (2016.01)

(21) Application number: **21180438.0**

(22) Date of filing: **18.06.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.06.2020   US 202063040988 P**
**17.07.2020   US 202063053316 P**
**03.11.2020   US 202017088013**
**14.08.2020   US 202016994349**

(71) Applicant: **Rockwell Collins, Inc.**
**Cedar Rapids, IA 52498 (US)**

(72) Inventors:
• **CHAPMAN, Anthony J.**
  **Annapolis, MD 21403 (US)**
• **AGRAWAL, Piyush**
  **South Windsor, CT 06074 (US)**
• **SHAPIRO, Jeffrey A.**
  **Cedar Rapids, IA 52402 (US)**

(74) Representative: **Dehns**
**St. Bride's House**
**10 Salisbury Square**
**London EC4Y 8JD (GB)**

(54) **CONTACT-LESS PASSENGER SCREENING AND IDENTIFICATION SYSTEM**

(57)    A contact-less passenger screening and identification system includes a temperature sensor (102), a blood oxygen saturation sensor, a cardiorespiratory sensor unit (104), one or more biometric sensor units (112), a communication circuitry (106), and one or more controllers (110). The one or more controllers may include one or more processors communicatively coupled to the temperature sensor, the cardiorespiratory sensor unit, and the one or more biometric sensor units. The one or more processors may be configured to execute a set of program instructions maintained in one or more memory units, where the set of program instructions is configured to cause the one or more processors to receive one or more signals, determine one or more physiological states of a subject (206), determine one or more identities of a subject, determine one or more business functions, and transmit one or more signals instructive of the one or more business functions.

FIG.2A

## Description

## CROSS-REFERENCE TO RELATED APPLCIATION

The present application claims the benefit of U.S. Provisional Application Serial Number 63/040,988, filed June 18, 2020, entitled PHYSIOLOGICAL STATE SCREENING SYSTEM, naming Anthony Chapman, Piyush Agrawal, and Geoffrey Shapiro as inventors, and U.S. Provisional Application Serial Number 63/053,316, filed July 17, 2020, entitled CONTACT-LESS PASSENGER SCREENING AND IDENTIFICATION SYSTEM, naming Anthony Chapman as inventor.

## TECHNICAL FIELD

The present disclosure relates generally to contact-less passenger screening and identification, and, more particularly, to the contact-less screening and identification of passengers of commercial aircraft.

## BACKGROUND OF THE INVENTION

Airlines face significant challenges-from both customer confidence and regulatory standpoints-as a result of certain contagious vectors that may spread from time to time. In particular, airlines face difficulty with respect to certain contagious vectors that spready by incidental contact or respiratory droplets. Therefore, it would be desirable to provide one or more systems configured to associate one or more measurement of passenger vital signs an identity of the passenger. In this regard, it would be desirable to provide one or more systems that may alert aircraft crew, airline staff, and/or airport ground crew of vital signs of one or more passengers that may be indicative of one or more contagious infections and/or the identity of any such one or more passengers so that adequate precautions (e.g., social distancing, denied boarding, aircraft diversion, medical attention, secondary screening, and the like) may be implemented.

## SUMMARY

A contact-less passenger screening and identification system is disclosed, in accordance with one or more embodiments of the present disclosure. In one embodiment, the contact-less passenger screening and identification system may include a temperature sensor, a blood oxygen saturation sensor, a cardiorespiratory sensor unit, one or more biometric sensor units, a communication circuitry, and one or more controllers having one or more processors communicatively coupled to the temperature sensor, the blood oxygen saturation sensor, the cardiorespiratory sensor unit, and the one or more biometric sensor units, wherein the one or more processors are configured to execute a set of program instructions maintained in one or more memory units, wherein the set of program instructions is configured to cause the one or more processors to: receive one or more signals from at least one of the temperature sensor, the blood oxygen saturation sensor, or the cardiorespiratory sensor unit indicative of one or more physiological states of a subject; receive one or more signals from the one or more biometric sensor units indicative of one or more biometric features of the subject; determine one or more physiological states of the subject based on the one or more signals indicative of the one or more physiological states of the subject; determine one or more identities of the subject based on the one or more signals indicative of one or more biometric features of the subject; determine one or more business functions based on the one or more physiological states and the one or more identities of the subject; and transmit one or more signals instructive of the one or more business functions.

A method for contact-less passenger screening and identification is disclosed. In one embodiment, the method for ambient physiological state screening comprises: receiving one or more signals from at least one of the temperature sensor, the blood oxygen saturation sensor, or the cardiorespiratory sensor unit indicative of one or more physiological states of a subject; receiving one or more signals from the one or more biometric sensor units indicative of one or more biometric features of the subject; determining one or more physiological states of the subject based on the one or more signals indicative of the one or more physiological states of the subject; determining one or more identities of the subject based on the one or more signals indicative of one or more biometric features of the subject; determining one or more business functions based on the one or more physiological states and the one or more identities of the subject; and transmitting one or more signals instructive of the one or more business functions.

It is to be understood that both the foregoing general description and the following detailed description are exemplary and explanatory only and are not necessarily restrictive of the present disclosure. The accompanying drawings, which are incorporated in and constitute a part of the characteristic, illustrate subject matter of the disclosure. Together, the descriptions and the drawings serve to explain the principles of the disclosure.

## BRIEF DESCRIPTION OF THE DRAWINGS

Implementations of the inventive concepts disclosed herein may be better understood when consideration is given to the following detailed description thereof. Such description makes reference to the included drawings, which are not necessarily to scale, and in which some features may be exaggerated and some features may be omitted or may be represented schematically in the interest of clarity. Like reference numerals in the drawings may represent and refer to the same or similar element, feature, or function. In the drawings:

FIG. 1A is a simplified block diagram illustrating a contact-less passenger screening and identification system, in accordance with one or more embodiments of the present disclosure.

FIG. 1B is a simplified block diagram illustrating a contact-less passenger screening and identification system, in accordance with one or more embodiments of the present disclosure.

FIGS. 2A and 2B are simplified conceptual views of a contact-less passenger screening and identification system, in accordance with one or more embodiments of the present disclosure.

FIG. 3 is a process flow diagram illustrating the sub-steps of a method of contact-less passenger screening and identification, in accordance with one or more embodiments of the present disclosure.

## DETAILED DESCRIPTION

Reference will now be made in detail to the subject matter disclosed, which is illustrated in the accompanying drawings.

Before explaining at least one embodiment of the inventive concepts disclosed herein in detail, it is to be understood that the inventive concepts are not limited in their application to the details of construction and the arrangement of the components or steps or methodologies set forth in the following description or illustrated in the drawings. In the following detailed description of embodiments of the instant inventive concepts, numerous specific details are set forth in order to provide a more thorough understanding of the inventive concepts. However, it will be apparent to one of ordinary skill in the art having the benefit of the instant disclosure that the inventive concepts disclosed herein may be practiced without these specific details. In other instances, well-known features may not be described in detail to avoid unnecessarily complicating the instant disclosure. The inventive concepts disclosed herein are capable of other embodiments or of being practiced or carried out in various ways. Also, it is to be understood that the phraseology and terminology employed herein is for the purpose of description and should not be regarded as limiting.

As used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only and should not be construed to limit the inventive concepts disclosed herein in any way unless expressly stated to the contrary.

In addition, use of the "a" or "an" are employed to describe elements and components of embodiments of the instant inventive concepts. This is done merely for convenience and to give a general sense of the inventive concepts, and "a' and "an" are intended to include one or at least one and the singular also includes the plural unless it is obvious that it is meant otherwise.

Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

Further, any arrangement of components to achieve a same functionality is effectively "associated" such that the desired functionality is achieved, such that any two components herein combined to achieve a particular functionality can be seen as "associated with" each other (irrespective of architectures or intermedial components). Any two components so associated can also be viewed as being "operably connected" or "operably coupled" to each other to achieve the desired functionality, and any two components capable of being so associated can also be viewed as being "operably couplable" to each other to achieve the desired functionality. Examples of operably couplable include, but are not limited to, physically mateable and/or physically interacting components, wirelessly interactable and/or wirelessly interacting components, logically interacting and/or logically interactable components, or the like.

Further, one or more components may be referred to herein as "configured to," "configurable to," "operable/operative to," "adapted/adaptable," "able to," "conformable/conformed to," etc. Those skilled in the art will recognize that such terms (e.g., "configured to") can generally encompass active-state components and/or inactive-state components and/or standby-state components, unless context requires otherwise.

Finally, as used herein any reference to "one embodiment," or "some embodiments" means that a particular element, feature, structure, or characteristic described in connection with the embodiment is included in at least one embodiment of the inventive concepts disclosed herein. The appearances of the phrase "in some embodiments" in various places in the specification are not necessarily all referring to the same embodiment, and embodiments of the inventive concepts disclosed may include one or more of the features expressly described or inherently present herein, or any combination of sub-combination of two or more such features, along with any other features which may not necessarily be expressly described or inherently present in the instant disclosure.

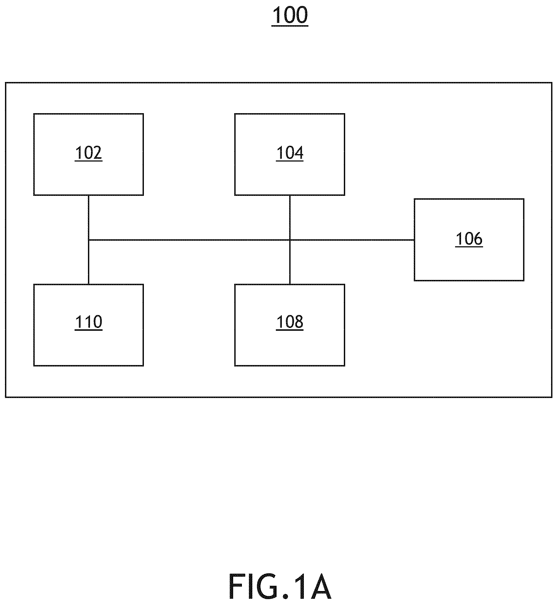

FIG. 1A illustrates a simplified block diagram illustrating a contact-less passenger screening and identification system 100 in accordance with one or more embodiments of the present disclosure. The contact-less passenger screening and identification system 100 may include a temperature sensor 102, a cardiorespiratory sensor unit 104, a communication circuitry 106, a memory unit 108, and one or more controllers 110 communicatively coupled to each of the temperature sensor 102 and the cardiorespiratory sensor unit 104. The one or more controllers 110 may include one or more processors. One or more components of the contact-less passenger screening and identification system 100 may be communicatively coupled to one or more other components of the contact-less passenger screening and identification system 100 in any manner known in the art. For example, the temperature sensor 102, the cardiorespiratory sensor unit 104, the communication circuitry 106, and the one or more controllers 110 may be communicatively coupled to each other and other components via a wireline connection (e.g., copper wire, fiber optic cable, soldered connection, and the like) or a wireless connection (e.g., RF coupling, IR coupling, data network communication (e.g., WiFi, WiMAX, Bluetooth, and the like)).

The temperature sensor 102 may include any sensor type known in the art to be suitable for measuring or determining the bodily temperature of a human subject within an ambient environment. For example, the temperature sensor 102 may include, but is not limited to, one or more infrared sensors, including an infrared sensor configured to include one or more focal plane arrays, one or more hyperspectral sensor units, and/or one or more thermal emission scanning units. As used herein, the term "ambient environment" may refer to, but is not limited to, communal spaces where it is typical for human subjects to congregate, including, without limitation, one or more locations within airports, aircrafts, public transportation, and the like.

The temperature sensor 102 may be configured to measure and/or determine the core bodily temperature of a human subject within an ambient environment. For example, the temperature sensor 102 may be configured to generate one or more signals (e.g., radiometric infrared images) indicative of bodily temperature based on a skin temperature of one or more human subjects. The one or more controllers 110 may be configured to direct the temperature sensor 102 to generate one or more signals indicative of the bodily temperature of a particular region of interest of the human body (e.g., the face of a human subject).

The cardiorespiratory sensor unit 104 may include any sensor type known in the art to be suitable for measuring or determining one or more cardiorespiratory states (e.g., breathing rate, respiration pattern, and the like) of a human subject within an ambient environment. For example, the cardiorespiratory sensor unit 104 may include any device configured to transmit electromagnetic waves from a transmitter and to receive electromagnetic waves at a receiver. In another example, the cardiorespiratory sensor unit 104 may include a radar sensor unit, including, without limitation, a radar sensor unit configured to transmit electromagnetic radiation from a transmitter and to receive electromagnetic radiation at a receiver. By way of an additional example, the cardiorespiratory sensor unit 104 may include any low-cost radar device known to be suitable to measure or determine one or more cardiorespiratory states of a human subject. As used herein, the term "ambient environment" may refer to, but is not limited to, communal spaces where it is typical for human subjects to congregate, including, without limitation, one or more locations within airports, aircrafts, public transportation, and the like.

The cardiorespiratory sensor unit 104 may be configured to measure and/or determine one or more cardiorespiratory states of a human subject within an ambient environment. For example, the cardiorespiratory sensor unit 104 may be configured to transmit one or more electromagnetic waves within an ambient environment containing one or more human subjects. The cardiorespiratory sensor unit 104 may further be configured to receive one or more electromagnetic waves following one or more interactions of the one or more electromagnetic waves with one or more human subjects.

The one or more controllers 110 may be configured to determine one or more cardiorespiratory states of the one or more human subjects based on one or more signals generated by the cardiorespiratory sensor unit 104. For example, the one or more controllers 110 may process one or more signals from the cardiorespiratory sensor unit 104 to determine one or more phase changes of the one or more electromagnetic waves transmitted by the cardiorespiratory sensor unit 104. In this regard, the one or more controllers 110 may determine minor movements of one or more portions (e.g., a chest or a heart) of the one or more human subjects, where the minor movements may be indicative of one or more cardiorespiratory states of the one or more human subjects. By way of an additional example, the one or more controllers 110 may be configured to determine one or more distance differentials between the cardiorespiratory sensor unit 104 and the one or more human subjects. In this regard, the cardiorespiratory sensor unit 104 may be configured to measure one or more signals indicative of respiration rate by comparing the difference between a first measured distance between the cardiorespiratory sensor unit 104 and one or more human subjects and a second measured distance between the cardiorespiratory sensor unit 104 and the one or more human subjects. Put another way, the cardiorespiratory sensor unit 104 may measure the distance between the contact-less passenger screening and identification system 100 and one or more human subjects at a first point in time at which at least one of the one or more human subjects inhales and at a second point in time at which the at least one human subject exhales. Accordingly, the one or more controllers 110 may be configured to determine one or more physiological states of the one or more human subjects using the following formula, where $D_{exhale}$ represents the distance between the contact-less passenger screening and identification system 100 and a human subject at the point in time at which the human subject exhales, and where $D_{inhale}$ represents the distance between the contact-less passenger screening and identification system 100 and the human subject at the point in time at which the human subject exhales:

$$\Delta D = D_{exhale} - D_{inhale}$$

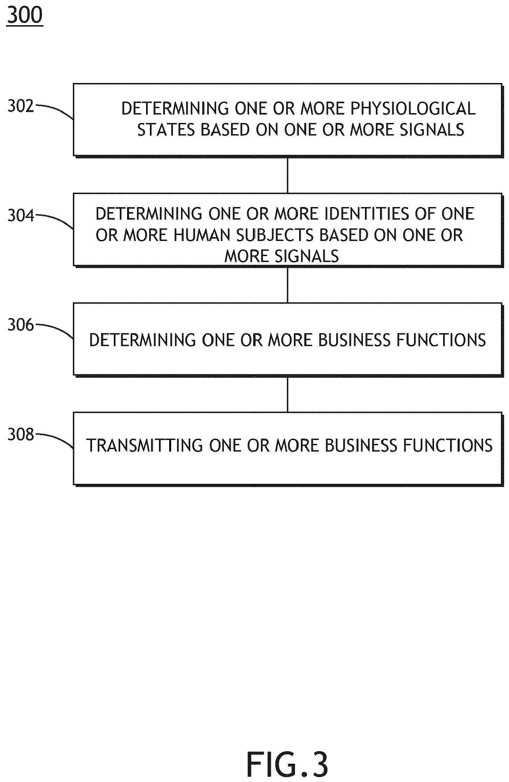

The one or more controllers 110 may be configured to generate one or more plots for display to a user via one or more user interfaces based on the one or more signals generated by at least one of the temperature sensor 102 or the cardiorespiratory unit 104 (see FIGS. 3A through FIGS. 3C and the accompanying description herein).

It is noted that the embodiments of the present disclosure are not limited to a cardiorespiratory sensor unit 104 or a temperature sensor 102, and that the contact-less passenger screening and identification system 100 may include any type of physiological sensing device known in the art. For example, the contact-less passenger screening and identification system 100 may include one or more sensors or devices configured to determine a blood oxygen saturation of one or more human subjects. In this regard, the one or more controllers 110 may be communicatively coupled to the physiological sensing device, and the physiological sensing device may perform one or more functions of the present disclosure via the one or more controllers 110.

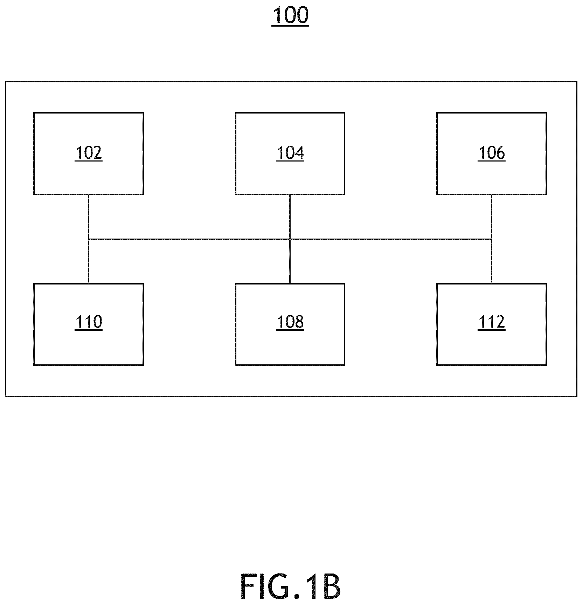

FIG. 1B illustrates a simplified block diagram illustrating a contact-less passenger screening and identification system 100 in accordance with one or more embodiments of the present disclosure. The contact-less passenger screening and identification system 100 may be configured to include one or more biometric sensor units 112 communicatively coupled to one or more components of the contact-less passenger screening and identification system 100. For example, the one or more biometric sensor units 112 may be communicatively coupled to one or more of the temperature sensor 102, the cardiorespiratory sensor unit 104, the communication circuitry 106, or the one or more controllers 110 via a wireline connection (e.g., copper wire, fiber optic cable, soldered connection, and the like) or a wireless connection (e.g., RF coupling, IR coupling, data network communication (e.g., WiFi, WiMAX, Bluetooth, and the like)). The one or more biometric sensor units 112 may include any device known in the art to be suitable for identification of a human subject using one or more biometric features of the human subject. For example, the one or more biometric sensor units 112 may include one or more cameras (e.g., RGB cameras and the like) configured for face recognition (e.g., for recognition via the one or more controllers 110 of one or more facial features and/or profiles of a known human subject stored in the memory unit 108). By way of additional example, the one or more biometric sensor units 112 may include one or more fingerprint sensors, one or more retinal and/or iris scanning sensors, or any other device configured to compare one or more collected signals indicative of a biometric feature of a human subject against one or more biometric features of the human subject stored in memory.

In an alternative embodiment, the contact-less passenger screening and identification system 100 may be communicatively coupled to one or more biometric sensor units 112 located in a remote location. For example, the contact-less passenger screening and identification system 100 may be connected via the communication circuitry 106 to one or more biometric sensor units 112 located at a user touch-point or interface.

The one or more processors of the one or more controllers 110 may include any one or more processing elements known in the art. In general, the term "processor" may be broadly defined to encompass any device having one or more processing elements, which execute program instructions from a non-transitory memory medium (i.e., memory). In one embodiment, the one or more processors may include any microprocessor-type computational device configured to execute software algorithms and/or instructions. In general, the processor may be broadly defined to encompass any device having data processing or logic capabilities. It should be recognized that the steps described throughout the present disclosure may be carried out by a single contact-less passenger screening and identification system 100 or by a plurality of contact-less passenger screening and identification systems 100.

The communication circuitry 106 may include any communication circuitry known in the art. The communication circuitry 106 may include one or more components that may be configured to transmit data in a manner that combines elements of any configuration of transmission known in the art. For instance, the communication circuitry 106 may include wireline-based communication circuitry (e.g., DSL-based interconnection, cable-based interconnection, T9-based interconnection, fiber-optic lines, and the like). In another instance, the communication circuitry 106 may include wireless-based communication circuitry, such as one or more of GSM, GPRS, CDMA, EV-DO, EDGE, WiMAX, 3G, 4G, LTE, 5G, 6G, Wi-Fi protocols, LoRa, customized RF protocol, and the like. The communication circuitry 106 may be configured to transmit one or more signals to a user interface

The memory unit 108 may include a memory unit or storage medium known in the art to be suitable for storing program instructions executable by the one or more processors of the one or more controllers 110. For example, the memory unit 108 may include a non-transitory memory medium. For instance, the memory unit 108 may include, but is not limited to, a read-only memory (ROM), a random-access memory (RAM), a magnetic or optical memory device, a magnetic tape, a solid-state drive, and the like. In another embodiment the memory unit 108 may be configured for storing one or more signals received from the temperature sensor 102 and/or the cardiorespiratory sensor unit 104. It is further noted that the memory unit 108 may be housed in a common housing with the one or more processors. In an alternative embodiment, the memory unit 108 may be located in a remote server. The memory unit 108 may maintain program instructions for causing the processor 114 to carry out various steps described in the present disclosure.

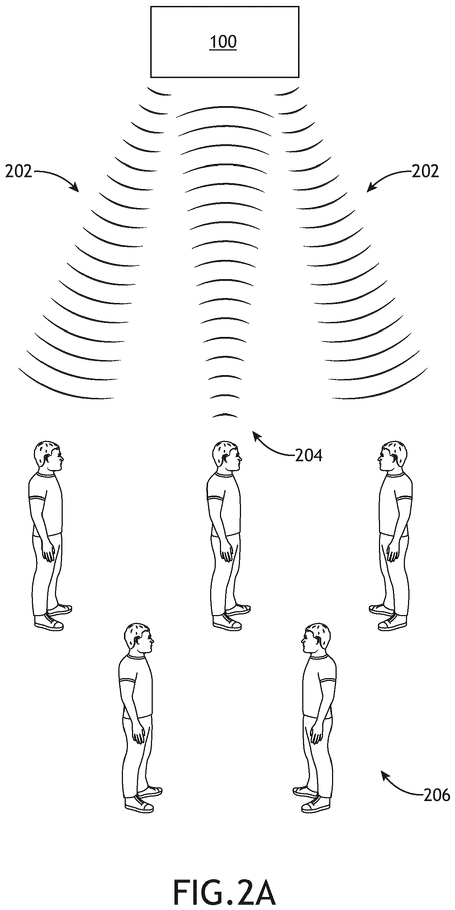

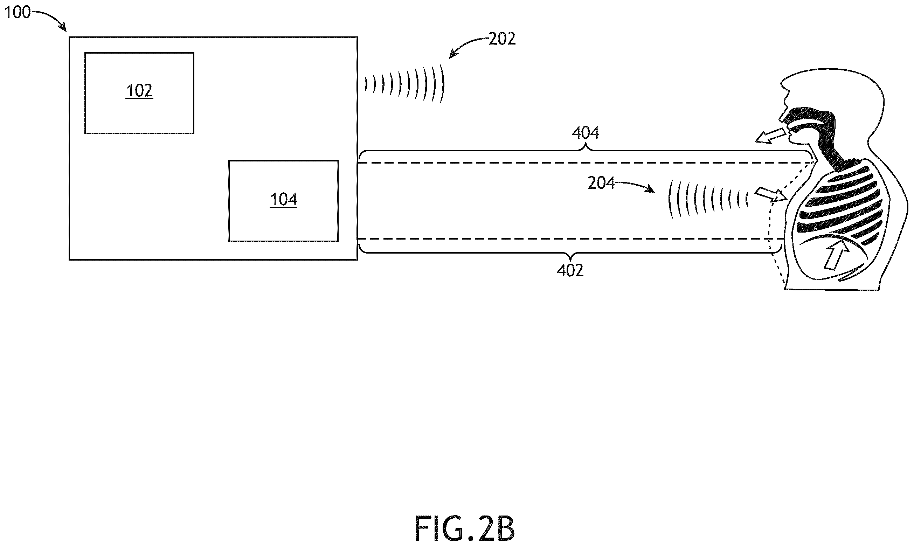

FIGS. 2A and 2B are simplified conceptual views of a contact-less passenger screening and identification system 100, in accordance with one or more embodiments of the present disclosure. The contact-less passenger screening and identification system 100 may be positioned within an ambient environment, including, without limitation, one or more portions of an airport, an aircraft, or any other location in which a plurality of human subjects 206 may congregate. The contact-less passenger screening and identification system 100 may transmit one or more sensing signals 202 via at least one of the temperature sensor 102 or the cardiorespiratory sensor unit 104, where the sensing signals 202 are directed toward the plurality of human subjects 206. The sensing signals 202 may include any electromagnetic wave, including, without limitation, one or more infrared waves or one or more radar waves. For example, the temperature sensor 102 may emit one or more infrared waves toward the plurality of human subjects 206. By way of an additional example, the cardiorespiratory sensor unit 104 may emit one or sensing signals 202 toward the plurality of human subjects 206. The contact-less passenger screening and identification system 100 may be configured to receive one or more reflected signals 204. For example, the one or more reflected signals 204 may be reflected by at least one of the plurality of human subjects 206 following interaction with one or more portions of the at least one of the plurality of human subjects 206. The one or more reflected signals 204 may include (or may be processed to generate) signals indicative of one or more physiological states of at least one of the plurality of human subjects 206.

In this regard, the contact-less passenger screening and identification system 100 (e.g., via the one or more controllers 110) may be configured to determine minor movements of one or more portions (e.g., a chest or a heart) of the plurality of human subjects 206, where the minor movements may be indicative of one or more cardiorespiratory states of the plurality of human subjects 206. By way of an additional example, the one or more controllers 110 may be configured to determine one or more distance differentials between the cardiorespiratory sensor unit 104 and the plurality of human subjects 206. In this regard, the cardiorespiratory sensor unit 104 may be configured to measure one or more signals indicative of respiration rate by comparing the difference between the first distance 404 and the second distance 402.

As an additional example, the contact-less passenger screening and identification system 100 may be configured to determine one or more core bodily temperatures of the plurality of human subjects 206. For example, the contact-less passenger screening and identification system 100 may be configured to transmit one or more infrared waves toward the plurality of human subjects 206 receive one or more reflected infrared waves.

The one or more reflected infrared waves may include (or may be processed to generate) signals indicative of one or more bodily temperatures of the plurality of human subjects 206.

It is noted that the contact-less passenger screening and identification system 100 may be configured to determine one or more correlations between one or more determined physiological states of the plurality of human subjects 206. For example, the contact-less passenger screening and identification system 100 may be configured to determine a location of one or more points of interest of the body of one of the plurality of human subjects 206, and to subsequently determine one or more physiological states of the one of the plurality of human subjects 206 at the one or more points of interest determined. By way of an additional example, the contact-less passenger screening and identification system 100 may be configured to determine the location of a head of a human subject (e.g., via a camera), and may determine the bodily temperature of the human subject at the human subject's head (e.g., via the temperature sensor 102).

The contact-less passenger screening and identification system 100 may be configured to determine one or more physiological states of the plurality of human subjects 206 at different points in time and at different locations in space. For example, the contact-less passenger screening and identification system 100 may be configured to determine one or more physiological states of one or more of the plurality of human subjects 206 as the one or more of the plurality of human subjects moves about an ambient space. By way of an additional example, the contact-less passenger screening and identification system 100 may be configured to affect the transmission of the one or more sensing signals 202 about an area to reach a human subject (e.g., via electronic radar steering, mechanical manipulation, and the like).

The one or more controllers 110 may make each of the foregoing determinations by comparing one or more received signals to known physiological patterns and algorithms (e.g., algorithms designed to identify inhalation patterns, exhalation patterns, heartbeat patterns, etc., based on previously collected and known physiological phenomena) stored in memory. The one or more controllers 110 may be configured to make one or more associations between the one or more physiological states of one or more of the plurality of human subjects 206 and the one or more collected signals indicative of a biometric feature of the one or more of the plurality of human subjects 206. For example, the one or more controllers 110 may associate one or more physiological states with one or more identities of the one or more of the plurality of human subjects 206. In this regard, the one or more controllers 110 may identify one of more of the plurality of human subjects 206 having one or more physiological states (e.g., physiological states indicative of infection, illness, and the like). The one or more controllers 110 may be configured to determine one or more business functions based on the one or more physiological states and/or the identity of a particular human subject. For example, the one or more controllers 110 may determine one or more actions required by regulation (e.g., federal, state, or local law, order, or recommendation related to a human subject displaying one or more physiological states indicative of infection, illness, and the like) or private, commercial policy (e.g., airline policy with respect to a human subject displaying one or more physiological states indicative of infection, illness, and the like).

FIG. 3 is a process flow diagram illustrating the substeps of a method 300 of contact-less passenger screening and identification, in accordance with one or more embodiments of the present disclosure.

In Step 302, one or more physiological states are determined. For example, the one or more physiological states may be determined based on the one or more signals. By way of additional example, at least one of the temperature sensor 102 or the cardiorespiratory sensor unit 104 may transmit one or more sensing signals 202 toward the plurality of human subjects 206. By way of an additional example, the contact-less passenger screening and identification system 100 may receive one or more reflected signals 204 reflected from one or more of the human subjects 206. By way of an additional example, the one or more controllers 110 may determine one or more physiological states based on the one or more reflected signals 204. By way of another example, the one or more controllers 110 may determine the difference between the first distance 404 and the second distance 402. In this regard, contact-less passenger screening and identification system 100 may determine one or more physiological states, including, without limitation, a respiration pattern of the plurality of human subjects 206. As an additional example, the one or more controllers 110 may determine one or more phase changes of at least one of the sensing signals 202 or the reflected signals 204. In this regard, the one or more controllers 110 may determine a cardiorespiratory pattern of the plurality of human subjects 206. As an additional example, the one or more controllers 110 may determine one or more bodily temperatures of the plurality of human subjects 206 based on one or more sensing signals 202 transmitted by the temperature sensor 102.

In Step 304, one or more identities of one or more of the plurality of human subjects 206 are determined. For example, the one or more biometric sensor units 112 may collect one or more signals indicative of one or more biometric features of a human subject. By way of additional example, the one or more controllers 110 may compare one or more of the collected signals indicative of a biometric feature of a human subject against one or more biometric features of the human subject stored in memory to determine one or more identities. It is specifically contemplated that the one or more biometric features of the human subject stored in memory may include any information concerning the human subject, including, without limitation, biometric information, biographic information (e.g., age, place of residence, etc.), demographic information (e.g., race, gender, etc.), and the like. For example, the one or more biometric features of the human subject may include drivers license, passport, identification card, and other information concerning the human subject (e.g., date of birth, place of residence, race, gender, weight, height, etc.).

In Step 306, one or more business functions are determined based on the one or more physiological states and the one or more identities of the plurality of human subjects 206. For example, the one or more controllers 110 may determine one or more precautionary actions (e.g., self-isolation, social distancing, aircraft diversion) based on the one or more physiological states of the plurality of human subjects 206. The one or more physiological interventions may include any appropriate act or series of acts intended to reduce the spread of a contagious vector among identified passengers, nonidentified passengers, and crewmembers of an aircraft. It is specifically contemplated that the one or more business functions may include, but are not limited to, compliance with laws and/or regulations concerning certain health matters. For example, the one or more business functions may include one or more precautionary actions e.g., self-isolation, social distancing, aircraft diversion) determined based on any combination of the one or more physiological states of the plurality of human subjects 206 and/or the one or more identities of the plurality of the human subjects 206. By way of another example, the one or more business functions may include one or more precautionary actions determined based on the combination of the one or more physiological states of the plurality of human subjects 206 and one or more biometric features (e.g., age of a subject) that may be indicative of a physiological state of the subject (e.g., illness). For the sake of clarity, it is specifically contemplated that the one or more controllers 110 may determine the one or more business functions based on a combination of signals (e.g., signals indicative of the one or more business functions and/or the one or more biometric features) that, when taken together, may indicate that a subject may carry disease (e.g., a disease resulting from viral or bacterial infection).

In Step 308, one or more signals instructive of the one or more business functions are transmitted. For example, the one or more controllers 110 (e.g., via the communication circuitry 106 or a user interface) may transmit the one or more signals based on the one or more determined physiological interventions to a user or remote location. In this regard, contact-less passenger screening and identification system 100 may be configured to alert a user (e.g., an aircraft crew member, airline staff person, and/or airport ground crew member) of the determined physiological intervention so that appropriate steps may be taken, including, for example, denied boarding, aircraft diversion for medical attention, or consultation with one or more ground-based medical professionals. By way of an additional example, the one or more controllers 110 may be configured to transmit one or more signals indicative of the one or more physiological interventions to a network or a remote server.

It is to be understood that embodiments of the methods in accordance with the inventive concepts disclosed herein may include one or more of the steps described herein. Further, such steps may be carried out in any desired order and two or more of the steps may be carried out simultaneously with one another. Two or more of the steps disclosed herein may be combined in a single step, and in some embodiments., one or more of the steps may be carried out as two or more sub-steps. Further, other steps or sub-steps may be carried in addition to, or as substitutes to one or more of the steps disclosed herein.

From the above description, it is clear that the inventive concepts disclosed herein are well adapted to carry out the objects and to attain the advantages mentioned herein as well as those inherent in the inventive concepts disclosed herein. While presently preferred embodiments of the inventive concepts disclosed herein have been described for purposes of this disclosure, it will be understood that numerous changes may be made which will readily suggest themselves to those skilled in the art and which are accomplished within the scope of the invention as defined by the claims.

**Claims**

1. A contact-less passenger screening and identification system, comprising:

   a temperature sensor 9102);
   a blood oxygen saturation sensor;
   a cardiorespiratory sensor unit (104);
   one or more biometric sensor units (112);
   a communication circuitry (106); and
   one or more controllers (110) having one or more processors communicatively coupled to the temperature sensor, the blood oxygen saturation sensor, the cardiorespiratory sensor unit, and the one or more biometric sensor units, wherein the one or more processors are configured to execute a set of program instructions maintained in one or more memory units, wherein the set of program instructions is configured to cause the one or more processors to:

   receive one or more signals from at least one of the temperature sensor, the blood oxygen saturation sensor, or the cardiorespiratory sensor unit indicative of one or more physiological states of a subject (206);
   receive one or more signals from the one or more biometric sensor units indicative of one or more biometric features of the subject;
   determine one or more physiological states of the subject based on the one or more signals indicative of the one or more physiological states of the subject;
   determine one or more identities of the subject based on the one or more signals indicative of one or more biometric features of the subject;
   determine one or more business functions based on the one or more physiological states and the one or more identities of the subject; and
   transmit one or more signals instructive of the one or more business functions.

2. The system of Claim 1, wherein the temperature sensor (102) comprises an infrared sensor.

3. The system of Claim 1 or 2, wherein the cardiorespiratory sensor unit comprises a radar unit.

4. The system of Claim 1, 2 or 3, wherein the one or more physiological states of the subject comprise at least one of a body temperature of the subject, a blood oxygen saturation of the subject, a heart rate of the subject, a respiration rate of the subject, or a respiration pattern of the subject.

5. The system of any preceding Claim, wherein the one or more biometric sensor units (112) comprise at least one of a camera configured for face recognition, one or more fingerprint sensors, one or more retinal scanning sensors, or one or more iris scanning sensors.

6. The system of any preceding Claim, wherein the system is communicatively coupled via the communication circuitry to at least one of one or more additional systems for ambient physiological state screening or one or more remote servers.

7. The system of any preceding Claim, wherein the one or more controllers are configured to determine one or more correlations between one or more physiological states of the subject.

8. A method for contact-less passenger screening and identification of a subject, comprising:

   receiving one or more signals from at least one of a temperature sensor, a blood oxygen saturation sensor, or a cardiorespiratory sensor unit indicative of one or more physiological states of a subject;
   receiving one or more signals from one or more biometric sensor units indicative of one or more biometric features of the subject;

determining one or more physiological states of the subject based on the one or more signals indicative of the one or more physiological states of the subject;
determining one or more identities of the subject based on the one or more signals indicative of one or more biometric features of the subject;
determining one or more business functions based on the one or more physiological states and the one or more identities of the subject; and transmitting one or more signals instructive of the one or more business functions.

9. The method of Claim 8, wherein the one or more physiological states of the subject comprise at least one of a body temperature of the subject, a blood oxygen saturation of the subject, a heart rate of the subject, a respiration rate of the subject, or a respiration pattern of the subject.

10. The method of Claim 8 or 9, further comprising determining one or more correlations between the one or more physiological states of the subject.

```
┌─────────────────────────────────────────────────────┐
│                                                       │
│   ┌─────────┐        ┌─────────┐                      │
│   │   102   │        │   104   │                      │
│   │         │        │         │      ┌─────────┐     │
│   └────┬────┘        └────┬────┘      │   106   │     │
│        │                  │           │         │     │
│        ├──────────────────┼───────────┘         │     │
│        │                  │           └─────────┘     │
│   ┌────┴────┐        ┌────┴────┐                      │
│   │   110   │        │   108   │                      │
│   │         │        │         │                      │
│   └─────────┘        └─────────┘                      │
│                                                       │
└─────────────────────────────────────────────────────┘
```

FIG.1A

EP 3 925 521 A1

FIG.1B

**FIG.2A**

FIG.2B

<u>300</u>

302 — DETERMINING ONE OR MORE PHYSIOLOGICAL STATES BASED ON ONE OR MORE SIGNALS

304 — DETERMINING ONE OR MORE IDENTITIES OF ONE OR MORE HUMAN SUBJECTS BASED ON ONE OR MORE SIGNALS

306 — DETERMINING ONE OR MORE BUSINESS FUNCTIONS

308 — TRANSMITTING ONE OR MORE BUSINESS FUNCTIONS

# FIG.3

Europäisches
Patentamt

European
Patent Office

Office européen
des brevets

# EUROPEAN SEARCH REPORT

**Application Number**

EP 21 18 0438

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2018/218286 A1 (SALTOR PTY LTD [AU]) 6 December 2018 (2018-12-06) * paragraphs [0036] - [0104] * * paragraphs [0342] - [0351] * * figure 1 * | 1-10 | INV. A61B5/00 A61B5/01 A61B5/0205 A61B5/113 A61B5/1172 A61B5/1171 |
| Y | US 2018/231653 A1 (PRADEEP VIVEK [US] ET AL) 16 August 2018 (2018-08-16) * paragraphs [0037] - [0068]; figures 1-8 * | 1-10 | |
| A | EP 3 441 783 A1 (SHAM WELLEN [TW]) 13 February 2019 (2019-02-13) * paragraphs [0018] - [0036] * | 1,3,9 | |
| A | WO 2019/068025 A1 (CHAPPELL ARVEL A [US]; OSTROVER LEWIS S [US]) 4 April 2019 (2019-04-04) * paragraphs [0046] - [0064] * | 7,10 | |
| A | WO 2018/031665 A1 (ELWHA LLC [US]) 15 February 2018 (2018-02-15) * paragraphs [0024] - [0026] * | 1-10 | TECHNICAL FIELDS SEARCHED (IPC) A61B |

The present search report has been drawn up for all claims

| Place of search | Date of completion of the search | Examiner |
|---|---|---|
| Berlin | 3 November 2021 | Dydenko, Igor |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**  EP 21 18 0438

This annex lists the patent family members relating to the patent documents cited in the above-mentioned European search report.
The members are as contained in the European Patent Office EDP file on
The European Patent Office is in no way liable for these particulars which are merely given for the purpose of information.

03-11-2021

| Patent document cited in search report | | | Publication date | Patent family member(s) | | Publication date |
|---|---|---|---|---|---|---|
| WO 2018218286 | A1 | | 06-12-2018 | NONE | | |
| US 2018231653 | A1 | | 16-08-2018 | CN | 110291489 A | 27-09-2019 |
| | | | | CN | 110291760 A | 27-09-2019 |
| | | | | CN | 110300946 A | 01-10-2019 |
| | | | | CN | 110301118 A | 01-10-2019 |
| | | | | CN | 110313152 A | 08-10-2019 |
| | | | | CN | 110313153 A | 08-10-2019 |
| | | | | CN | 110313154 A | 08-10-2019 |
| | | | | CN | 110326041 A | 11-10-2019 |
| | | | | CN | 110326261 A | 11-10-2019 |
| | | | | CN | 110383235 A | 25-10-2019 |
| | | | | EP | 3583485 A1 | 25-12-2019 |
| | | | | EP | 3583489 A1 | 25-12-2019 |
| | | | | EP | 3583497 A1 | 25-12-2019 |
| | | | | EP | 3583595 A1 | 25-12-2019 |
| | | | | EP | 3583746 A1 | 25-12-2019 |
| | | | | EP | 3583747 A1 | 25-12-2019 |
| | | | | EP | 3583748 A1 | 25-12-2019 |
| | | | | EP | 3583749 A1 | 25-12-2019 |
| | | | | US | 2018231653 A1 | 16-08-2018 |
| | | | | US | 2018232201 A1 | 16-08-2018 |
| | | | | US | 2018232563 A1 | 16-08-2018 |
| | | | | US | 2018232608 A1 | 16-08-2018 |
| | | | | US | 2018232645 A1 | 16-08-2018 |
| | | | | US | 2018232662 A1 | 16-08-2018 |
| | | | | US | 2018232902 A1 | 16-08-2018 |
| | | | | US | 2018233132 A1 | 16-08-2018 |
| | | | | US | 2018233139 A1 | 16-08-2018 |
| | | | | US | 2018233140 A1 | 16-08-2018 |
| | | | | US | 2018233141 A1 | 16-08-2018 |
| | | | | US | 2018233142 A1 | 16-08-2018 |
| | | | | US | 2018233145 A1 | 16-08-2018 |
| | | | | US | 2020012906 A1 | 09-01-2020 |
| | | | | US | 2020042839 A1 | 06-02-2020 |
| | | | | US | 2020104653 A1 | 02-04-2020 |
| | | | | WO | 2018151979 A1 | 23-08-2018 |
| | | | | WO | 2018151980 A1 | 23-08-2018 |
| | | | | WO | 2018152006 A1 | 23-08-2018 |
| | | | | WO | 2018152007 A1 | 23-08-2018 |
| | | | | WO | 2018152008 A1 | 23-08-2018 |
| | | | | WO | 2018152009 A1 | 23-08-2018 |
| | | | | WO | 2018152010 A1 | 23-08-2018 |
| | | | | WO | 2018152011 A1 | 23-08-2018 |
| | | | | WO | 2018152012 A1 | 23-08-2018 |
| | | | | WO | 2018152013 A1 | 23-08-2018 |

EPO FORM P0459

For more details about this annex : see Official Journal of the European Patent Office, No. 12/82

page 1 of 2

**EP 3 925 521 A1**

**ANNEX TO THE EUROPEAN SEARCH REPORT
ON EUROPEAN PATENT APPLICATION NO.**                    EP 21 18 0438

This annex lists the patent family members relating to the patent documents cited in the above-mentioned European search report.
The members are as contained in the European Patent Office EDP file on
The European Patent Office is in no way liable for these particulars which are merely given for the purpose of information.

03-11-2021

| Patent document cited in search report | | Publication date | Patent family member(s) | | Publication date |
|---|---|---|---|---|---|
| | | | WO | 2018152014 A1 | 23-08-2018 |
| | | | WO | 2018152016 A1 | 23-08-2018 |
| EP 3441783 | A1 | 13-02-2019 | CN | 109381220 A | 26-02-2019 |
| | | | EP | 3441783 A1 | 13-02-2019 |
| | | | US | 2019046120 A1 | 14-02-2019 |
| WO 2019068025 | A1 | 04-04-2019 | CN | 111742560 A | 02-10-2020 |
| | | | CN | 111758229 A | 09-10-2020 |
| | | | CN | 111936036 A | 13-11-2020 |
| | | | EP | 3687388 A1 | 05-08-2020 |
| | | | EP | 3688897 A1 | 05-08-2020 |
| | | | EP | 3688997 A1 | 05-08-2020 |
| | | | KR | 20200127150 A | 10-11-2020 |
| | | | KR | 20200127969 A | 11-11-2020 |
| | | | KR | 20200130231 A | 18-11-2020 |
| | | | US | 2020296458 A1 | 17-09-2020 |
| | | | US | 2020296480 A1 | 17-09-2020 |
| | | | US | 2020297262 A1 | 24-09-2020 |
| | | | WO | 2019067783 A1 | 04-04-2019 |
| | | | WO | 2019068025 A1 | 04-04-2019 |
| | | | WO | 2019068035 A1 | 04-04-2019 |
| WO 2018031665 | A1 | 15-02-2018 | CN | 109843175 A | 04-06-2019 |
| | | | EP | 3496609 A1 | 19-06-2019 |
| | | | JP | 2019535050 A | 05-12-2019 |
| | | | SG | 11201901031R A | 28-03-2019 |
| | | | WO | 2018031665 A1 | 15-02-2018 |

EPO FORM P0459

For more details about this annex : see Official Journal of the European Patent Office, No. 12/82

**EP 3 925 521 A1**

REFERENCES CITED IN THE DESCRIPTION

**Patent documents cited in the description**

- US 63040988

- US 63053316